(19) **Europäisches Patentamt**
**European Patent Office**
**Office européen des brevets**

(11) **EP 3 884 848 A1**

(12) **EUROPEAN PATENT APPLICATION**

(43) Date of publication:
**29.09.2021 Bulletin 2021/39**

(51) Int Cl.:
**A61B 5/00** *(2006.01)* **A61B 5/02** *(2006.01)*
**A61B 6/00** *(2006.01)*

(21) Application number: **20164757.5**

(22) Date of filing: **23.03.2020**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Kardiolytics Inc.**
**Tulsa, OK 74114 (US)**

(72) Inventors:
• **SIEMIONOW, Kris B.**
**Chicago, IL Illinois 60610 (US)**

• **LEWICKI, Paul**
**Tulsa, OK Oklahoma 74114 (US)**
• **SADOWSKI, Wojciech**
**41-813 Zabrze (PL)**
• **MALOTA, Zbigniew**
**43-100 Tychy (PL)**
• **KRAFT, Marek**
**01-949 Warszawa (PL)**

(74) Representative: **Kancelaria Eupatent.pl Sp. z.o.o**
**Ul. Kilinskiego 185**
**90-348 Lodz (PL)**

(54) **A SYSTEM AND A METHOD FOR DETERMINING A SIGNIFICANCE OF A STENOSIS**

(57) A method for determining a significance of a stenosis in a currently examined blood vessel, the method comprising: providing a pre-trained reasoning module (130) that has been trained to output a value of a stenosis significance parameter by means of a training data set comprising a plurality of records of prior clinically examined stenosis cases, each training record comprising data related to dimensional parameters, blood flow parameters and clinical measurement parameters of the prior clinically examined blood vessel containing the stenosis; inputting, to the pre-trained reasoning module (130), an examination record comprising data related to the dimensional parameters of the currently examined blood vessel containing the stenosis and instructing the reasoning module (130) to output the value of the stenosis significance parameter based on the examination record.

Fig. 1

**Description**

TECHNICAL FIELD

The present invention relates to a system and a method for determining a significance of a stenosis by means of an autonomous, artificial intelligence system.

BACKGROUND

A stenosis is an abnormal narrowing in a blood vessel. It can be caused by a lesion that reduces the space of lumen of the blood vessel.

The significance of the narrowing depends on both the shape, degree of narrowing and its location. The most significant are the narrowing of the main branches. Coronary artery disease (CAD) is most often associated with the narrowing or blockage of blood vessels by the atherosclerotic plaque, which can lead to a decrease in blood supply, and thus oxygen, to the heart muscle. Atherosclerotic plaque most often forms at specific sites of the artery, such as arterial bends or bifurcation.

A very important aspect in the prevention and treatment of CAD is the functional evaluation of narrowed or blocked blood vessels. Stable forms of coronary heart disease often do not require treatment other than pharmacological treatment. However, in many cases revascularization is necessary by means of either percutaneous angioplasty or coronary artery bypass grafting. The decision about revascularization in relation to intermediate stenosis, when reduction of the diameter of the artery light is in the range from 50% to 70%, is particularly controversial. It is estimated that about half of them are hemodynamically significant, although they are often overlooked at revascularization.

Currently, the basic methods for assessing the hemodynamic significance of coronary stenosis are cardiac stress tests, contrast angiography, and measuring the fractional flow reserve (FFR). Practically, all these methods have some limitations and disadvantages.

The exercise stress test allows the assessment of the heart's function in response to increased physical effort and increased oxygen demand. It is a stress test and therefore, there are numerous contraindications to its performance, such as: very advanced ischemic heart disease, advanced aortic valve disease, severe heart failure, acute infection, recent stroke, unstabilized arterial hypertension and osteoarthritis.

In diagnostic imaging the stenosis significance is typically assessed based on two-dimensional images. The spatial shape of the plaque, its location and its influence on flow disturbances as well energy losses are completely neglected.

The measurement of FFR is, in turn, an invasive examination performed during angiography. Additionally, non-linear relationship between pressure-flow, the influence of external vessel forces, size of the perfusion area, microvascular dysfunction as well as flow disorders induced by the catheter placed into vessel can affect the accuracy of the FFR assessment. The FFR measurement requires maximum coronary flow. However, this cannot be completely achieved by administration of adenosine (an expanding agent). The use of FFR requires a deep knowledge of anatomy, physiology and pathophysiology of coronary arteries. Many factors influence the accuracy with which FFR estimates stenosis-induced flow impairment in the individual patient. The coronary reserve depends on hemodynamics, especially pressure and heart rate as well depends on age, gender and health condition. In addition, the non-linear relationship between pressure-flow, the influence of external vessel forces, size of the perfusion area, microvascular dysfunction as well as flow disorders induced by the catheter placed into vessel can also affect the accuracy of FFR assessment.

Assessment of the hemodynamic significance of the stenosis show 30-40 % discordance between FFR, defined as the ratio of mean distal coronary pressure to mean aortic pressure, and CFR, defined as the ratio of maximal flow to flow at rest at the same perfusion pressure.

However, FFR has a statistically significant correlation with the aortic pressure in coronary lesions. In addition, the index calculated in this way depends on the diameter of the vessel. A small, healthy blood vessel will have a similar value of FFR as a large, narrowed vessel with the same active lumen of artery. Combining simultaneous pressure and flow velocity information, may increase the effectiveness of assessing the significance of arterial stenosis. The degree of narrowing does not transfer easily into the functional significance of the change.

SUMMARY OF THE INVENTION

There is a need for alternative methods of determining the significance of the stenosis.

The invention relates to a method for determining a significance of a stenosis in a currently examined blood vessel, the method comprising: providing a pre-trained reasoning module that has been trained to output a value of a stenosis significance parameter by means of a training data set comprising a plurality of records of prior clinically examined stenosis cases, each training record comprising data related to dimensional parameters, blood flow parameters and clinical measurement parameters of the prior clinically examined blood vessel containing the stenosis; inputting, to the pre-trained reasoning module, an examination record comprising data related to the dimensional parameters of the currently examined blood vessel containing the stenosis and instructing the reasoning module to output the value of the stenosis significance parameter based on the examination record.

The examination record may further comprise data related to the blood flow parameters of the currently examined blood vessel containing the stenosis.

The dimensional parameters of the prior clinically examined blood vessel and/or of the currently examined blood vessel may include at least one of: a length of the stenosis, a thickness of the stenosis, a shape index of the stenosis, a symmetry index of the stenosis, a cross-section area of a lesion coronary, an area severity of the stenosis, a stenosed vessel volume, a reconstructed vessel volume lumen, a volume severity of the stenosis, an aggregate volume of the stenosis, an aggregate volume severity of the stenosis, a coronary score lesion grade, a vascular index, an extent score, a calcium score, a density factor, a localization of the stenosis.

The blood flow parameters of the prior clinically examined blood vessel and/or of the currently examined blood vessel may include at least one of: relative fractional flow reserve ($FFR_{VCAST}$), energy flow reference index ($EFR_{VCAST}$), a vascular resistance (R), a turbulent kinetic energy, a pressure drop (DP), a wall shear stress, oscillatory shear index, a relative residence time.

The clinical measurement parameters of the prior clinically examined blood vessel may include at least one of: a fractional flow reserve, an instantaneous wave-free ratio, a resting full-cycle ratio or relative flow reserve.

The reasoning module may be a convolutional neural network.

The method may further comprise providing an image segmenter implemented as a neural network trained to process input image data and to output a 3D segmented model containing description of blood vessels arrangement within the imaged volume, including at least the location and dimensions of the vessels, wherein the image segmenter is trained by performing the following steps: prediction of output binary mask based on the input CT imaging data; the computation of the difference between the ground truth mask (as given in the training data) and the predicted mask; the update of weights according to the gradient backpropagation method.

The method may further comprise providing an image segmenter configured to process input image data and to output a 3D segmented model containing description of blood vessels arrangement within the imaged volume, including at least the location and dimensions of the vessels by use of at least one of the following half-automated or fully-automated methods: region growing from seed, active shapes and active contours, segmentation based on graph cuts, adaptive thresholding, segmentation based on rough sets, watershed segmentation, vesselness filters (Frangi, Jerman), connectedness methods (fuzzy, Bayesian).

The invention also relates to a computer-implemented system comprising: a pre-trained reasoning module that has been trained to output a value of a stenosis significance parameter by means of a training data set comprising a plurality of records of prior clinically examined stenosis cases, each training record comprising data related to dimensional parameters, blood flow parameters and clinical measurement parameters of the prior clinically examined blood vessel containing the stenosis; wherein the pre-trained reasoning module has an input configured to receive an examination record comprising data related to the dimensional parameters of the currently examined blood vessel containing the stenosis and an output configured to provide the value of the stenosis significance parameter based on the examination record.

These and other features, aspects and advantages of the invention will become better understood with reference to the following drawings, descriptions and claims.

BRIEF DESCRIPTION OF DRAWINGS

Various embodiments are herein described, by way of example only, with reference to the accompanying drawings, wherein:

Fig. 1 shows a functional diagram of the system in accordance with an embodiment of the invention in its training configuration.

Fig. 2 shows a functional diagram of the system in accordance with an embodiment of the invention in its first inference configuration.

Fig. 3 shows a functional diagram of the system in accordance with an embodiment of the invention in its second inference configuration.

Fig. 4 shows schematically a convolutional neural network.

DETAILED DESCRIPTION OF THE INVENTION

The following detailed description is of the best currently contemplated modes of carrying out the invention.

The description is not to be taken in a limiting sense, but is made merely for the purpose of illustrating the general principles of the invention.

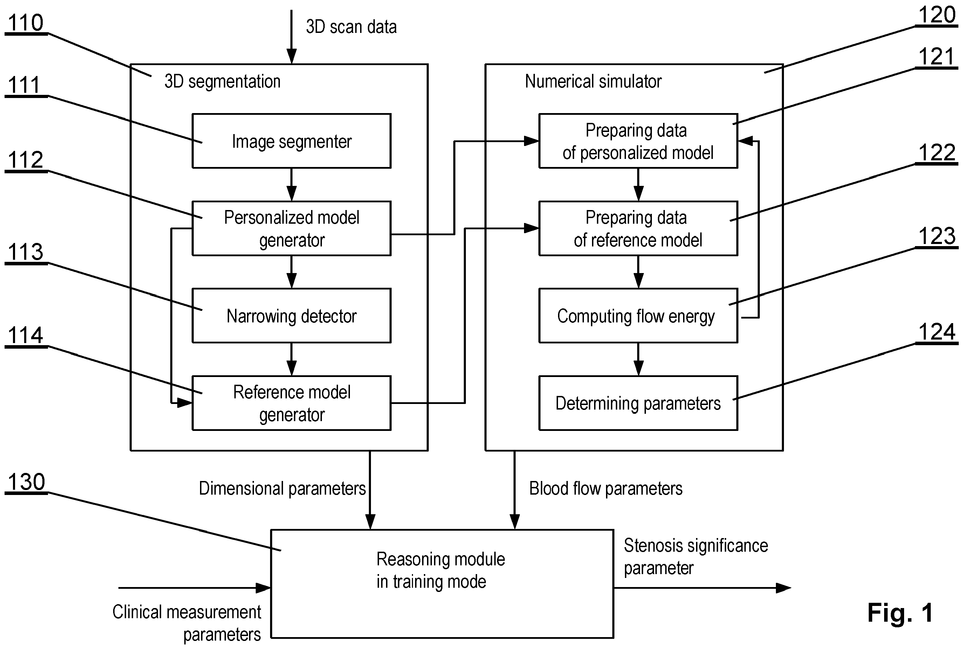

Fig. 1 shows a functional diagram of the system in accordance with an embodiment of the invention in its training configuration (in case it is implemented as a system to be trained, such as a convolutional neural network).

All components of the system can be controlled by one or more computers which is controlled by an operating system and one or more software applications. The computer may be equipped with a suitable memory which may store computer program or programs executed by the computer in order to execute steps of the methods utilized in the system. Computer programs are preferably stored on a non-transitory medium. An example of a non-transitory medium is a non-volatile memory, for example a flash memory while an example of a volatile memory is RAM. The computer instructions are executed by a processor. These memories are exemplary recording media for storing computer programs comprising computer-executable instructions performing all the steps of the computer-implemented method according the technical concept presented herein. Communication between the computers and the components of the system may be performed by wire or wirelessly, according to known communication means.

The main module of the system is a reasoning module 130 that may be implemented as a neural network, such as a convolutional neural network.

The reasoning module is pre-trained to output a value of a stenosis significance parameter by means of a training data set that contains records of prior clinically examined stenosis cases. Each training record comprises data related to:

- dimensional parameters of the blood vessel containing the stenosis, which can be input directly or determined by a 3D segmentation module 110 as described below;
- blood flow parameters of the blood vessel containing the stenosis, which can be input directly or determined by a numerical simulator module 120 as described below;
- clinical measurement parameters, which are determined as results of clinical examinations performed (such as invasive examinations), as described below.

The reasoning module is trained to determine the value of the stenosis significance parameter for an examination record containing data of a currently examined stenosis, wherein the examination record contains less input data than the training records - in particular, wherein the examination record contains only the dimensional parameters and optionally the blood flow parameters, but does not contain clinical measurement parameters. This will allow to determine (at least with some degree of probability) the stenosis significance on the basis of 3D scan data only, without the need to perform invasive clinical examinations.

A 3D segmentation module 110 may be provided for receiving 3D imaging data of blood vessels and generating a 3D segmented model of the blood vessels describing dimensional parameters. Any type of module can be used to provide this functionality and the module described herein is by way of example only.

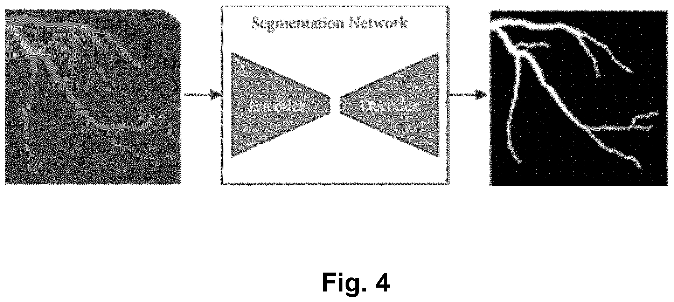

In one embodiment, the 3D segmentation module 110 may contain an image segmenter 111, for example based on a convolutional neural network (such as shown in Fig. 4) trained to process input image data (such as 3D computer tomography scans) and to output a 3D segmented model, containing description of blood vessels arrangement within the imaged volume, including at least the location and dimensions (such as diameter, cross-sectional lumen) of the vessels.

The convolutional neural network (CNN) for segmentation can be a class of deep, feed-forward artificial neural networks. The neural network preferably follows a U-Net like encoder-decoder architecture with shortcut connections as shown in Fig. 4.

To facilitate the use of transfer learning, the encoder part might be composed of a canonical neural network architecture such as VGG, Inception, ResNet or EfficientNet and a complementary decoder. Both the encoder and the complementary decoder might be of the 2D or the 3D variant.

Prior to its use for prediction and segmentation, the neural network must be trained. The training is performed using pairs of corresponding CT-segmentation mask volumes or regions of interest. The training data can have the form of a set of full corresponding CT-segmentation mask images (for the 2D variant) or volumes or volumetric regions of interest with their corresponding segmentation masks (for the 3D variant). During training, the network repeatedly performs the following steps:

- the step of prediction of output binary mask based on the input CT imaging data,
- the computation of the difference between the ground truth mask (as given in the training data) and the predicted mask
- the update of weights according to the gradient backpropagation method.

A fully trained neural network is capable of performing binary mask predictions, which are subsequently combined into 3D segmentation of coronary vessels.

The 2D variant performs predictions on a selection of 2D images (slices) taken from the complete 3D scan at different axes and angles. The final prediction is a 3D model of the coronary arteries composed by combining those individual predictions by voting, averaging or nonlinear filtering. The 3D variant operates directly on the volume data either as a whole, or represented by a set of regions of interest. Operation on the whole volume yields a complete 3D coronary artery segmentation. Operation on regions of interest yields partial 3D predictions, which are then concatenated or otherwise combined into the final 3D segmentation.

The image segmenter 111 may be also implemented manually (by hand annotation of coronary vessels based on 3D CT data).

Furthermore, the image segmenter 111 may be implemented by half-automated or fully-automated methods, such as:

- region growing from seed
- active shapes and active contours
- segmentation based on graph cuts
- adaptive thresholding
- segmentation based on rough sets
- watershed segmentation
- vesselness filters (Frangi, Jerman)
- connectedness methods (fuzzy, Bayesian)

In addition, the 3D segmentation module 110 may contain further blocks allowing provision of further data based on analysis of parameters of the blood vessels. For example, it may contain a narrowing detector 113 for identifying narrowings of the blood vessels, configured to detect narrowings along the blood vessels and provide their location and shape parameters.

Furthermore, model generators 112 and 114 may be used to generate personalized and reference blood vessel models, respectively. The model generators 112, 114 may be configured to operate according to the method disclosed in a patent application EP19174972, wherein the personalized model is based on the medical imaging data and the reference model is to reflect a state of healthy blood vessels that lack lesions based on the medical imaging data or based on numerical reconstruction of the personalized model.

In general, the 3D segmentation module may output a 3D segmented model that describes the blood vessels by various physical parameters, such as described below.

A length of the stenosis may be provided that defines the distance between the proximal and distal ends of the coronary lesion.

A thickness of the stenosis may be provided that defines the maximal distance between bottom and top (apex) of the stenosis.

A shape index of the stenosis may be provided that defines the relationship of the length of the stenosis to the thickness of the stenosis.

A symmetry (concentricity) index may be provided that defines the ratio of the length of the stenosis perimeter lying on the reconstructed wall to the total circumference of the reconstructed vessel at the stenosis site.

A cross-section area of lesion coronary may be provided that defines the minimal coronary artery lumen in stenosis place.

A cross-section area of reconstructed coronary may be provided that defines the maximal coronary artery lumen for reconstructed stenosis in the same place.

An area severity of the stenosis (stenosis %) may be provided that defines the Cross-section area of the lesion coronary to the cross-section area of the reconstructed coronary rate (multiplied by 100%). In addition, it can be divided into 4 groups, using the Gensini score: 1 for 1%-25 %; 2 for 26%-50 %; 4 for 51%-75%; 8 for 76%-90%; 16 for 91%-99%; and 32 for total occlusion.

A stenosed vessel volume (measured in $mm^3$) may be provided that defines a total volume of the lumen between the proximal and the distal ends of the coronary lesion.

A reconstructed vessel volume lumen (measured in $mm^3$) may be provided that defines the total volume of the stenosis between the proximal and the distal ends of the coronary lesion.

A volume severity of the stenosis may be provided that defines the ratio of the stenosis volume to the reconstructed lumen volume of vessel rate (multiplied by 100%).

An aggregate volume of the stenosis (or reconstruction models) may be provided.

An aggregate volume severity of the stenosis may be provided.

A coronary score lesion grade may be provided that defines: 1 - no lesions; 2 - lesions <50% area stenosis; 3-single lesion >50% but <90%; 4 - multiple lesions >50% but <90%; 4a - <3 vessel reference diameters - this case should be described as one lesion; 4b - >3 vessel reference diameters - this case should be described as two lesions; 5 - lesions >90% but <100%; 6 - 100%.

A vascular index may be provided that defines: 0 - no lesion; 1 - one-vascular stenosis; 2 - two-vascular stenosis; 3 - three-vascular stenosis; 4 - LM stenosis; 5 - LM and RCA stenosis.

An extent score may be provided that defines: 1 - when the lesions cover 10% of the branches segment's length; 2 - when the lesions cover 10-50% of the branches segment's length; 3- when the lesions cover > 50% 10% of the branches segment's length.

A calcium score may be provided that defines the weighted density score given to the highest attenuation value (HU) multiplied by the area of the calcification speck. The density factor may be: 130-199 HU: 1; 200-299 HU: 2; 300-399 HU: 3; 400+ HU: 4. For example, if a calcified speck has a maximum attenuation value of 400 HU and occupies 8 mm$^2$ area, then its calcium score will be 32. The score of every calcified speck is summed up to give the total calcium score. The grading of coronary artery disease (based on total calcium score) may be: 0 indicating no evidence of CAD; 1-10 indicating minimal disease; 11-100 indicating mild disease; 101-400 indicating moderate disease; >400 indicating severe disease.

A localization of the stenosis may be provided that defines:

*1. RCA proximal* - from ostium to one half the distance to the acute margin of the heart.
*2. RCA mid* - from end of first segment to acute margin of heart.
*3. RCA distal* - from the acute margin of the heart to the origin of the posterior descending artery.
*4. Posterior descending* - running in the posterior interventricular groove.
*16. Posterolateral from RCA* -posterolateral branch originating from the distal coronary artery distal to the crux.

> *a. Posterolateral from RCA* - first posterolateral branch from segment 16.
> *b. Posterolateral from RCA* - second posterolateral branch from segment 16.
> *c. Posterolateral from RCA* - third posterolateral branch from segment 16.

*5. Left main* - from the ostium of the LCA through bifurcation into left anterior descending and left circumflex branches.
*6. LAD proximal* - proximal to and including first major septal branch.
*7. LAD mid* - LAD immediately distal to origin of first septal branch and extending to the point where LAD forms an angle (RAO view). If this angle is not identifiable this segment ends at one half the distance from the first septal to the apex of the heart.
*8. LAD apical* - terminal portion of LAD, beginning at the end of previous segment and extending to or beyond the apex.
*9. First diagonal* -the first diagonal originating from segment 6 or 7:

> *a. First diagonal* - additional first diagonal originating from segment 6 or 7, before segment 8.

*10. Second diagonal* - second diagonal originating from segment 8 or the transition between segment 7 and 8.

> *a. Second diagonal a* - additional second diagonal originating from segment 8.

*11. Proximal circumflex* - main stem of circumflex from its origin of left main to and including origin of first obtuse marginal branch.
*12. Intermediate/anterolateral* - branch from trifurcating left main other than proximal LAD or LCX. Belongs to the circumflex territory.

> *a. Obtuse marginal a* - first side branch of circumflex running in general to the area of obtuse margin of the heart.
> b. *Obtuse marginal b* - second additional branch of circumflex running in the same direction as 12.

*13. Distal circumflex* - the stem of the circumflex distal to the origin of the most distal obtuse marginal branch and running along the posterior left atrioventricular grooves. Caliber may be small or artery absent.
14. *Left posterolateral* - running to the posterolateral surface of the left ventricle. May be absent or a division of obtuse marginal branch.

> *a. Left posterolateral a* - distal from 14 and running in the same direction.
> *b. Left posterolateral b* - distal from 14 and 14 a and running in the same direction.

*15. Posterior descending* -most distal part of dominant left circumflex when present. Gives origin to septal branches. When this artery is present, segment 4 is usually absent.

A numerical simulator 120 may be provided for calculating blood flow parameters associated with the 3D model generated by the 3D segmentation module 110.

In one embodiment, the numerical simulator 120 may contain computing blocks 121-124 for determining flow parameters according to the method disclosed in a patent application EP19174972. Namely, the computing blocks 121, 122 may prepare data (namely, inlet and outlet boundary and initial conditions of blood flow) of the personalized and reference blood vessels models obtained from model generators 112, 114. The computing block 123 may perform a numerical simulation of blood flow for the same physical and boundary conditions in the personalized model and in the reference model, the simulation comprising determining conditions of blood flow at an inlet to the blood vessels model and calculating blood flow energy for the inlet and all outlets of the blood vessels model. Finally, the block 124 may compare the blood flow energy measured for the personalized model and for the reference model and to determine flow energy change indexes of the blood flow in the personalized model and in the reference model.

For example, the numerical simulator 120 may calculate and output an energy flow reference index (EFR$_{VCAST}$) parameter that takes values less than 1 and greater than 0, wherein the more the value of this parameter is closer to 0, the greater is the hemodynamic significance of the stenosis in a given branch of coronary.

The numerical simulator 120 may also calculate other parameters such as vascular resistance R, turbulent kinetic energy (TKE), pressure drop (DP), wall shear stress (WSS), oscillatory shear index (OSI) or relative residence time (RRT), as described in details in European patent application EP19174972.

The clinical examinations data may include the following parameters, determined as a result of invasive examinations.

A fractional flow reserve (FFR) may be determined by clinical invasive measurement - as a ratio of the maximum coronary flow in the presence of a stenosis (Qs) to the maximum flow in a vessel without stenosis (Qn). It can be determined as the ratio of the average pressure measured after the stenosis (P$_d$) in the distal section of the vessel to the average pressure in the aorta (P$_a$) in the conditions of maximum hyperemia (assuming that central venous pressure (Pv) is much lower than Pa and Pd):

$$FFR = \frac{Qs}{Qn} = \frac{(P_d - P_v)/R}{(P_a - P_v)/R} = \frac{P_d}{P_a}$$

where $P_a$ is the pressure in the aorta, $P_v$ is the central venous pressure, and $P_d$ is the pressure after the stenosis.

An instantaneous wave-free ratio (iFR) may be determined as a pressure gradient across a stenosis during the wave-free period, when resistance is constant and minimalized in the cardiac cycle. It can be determined as the ratio of distal coronary pressure (Pd) to the pressure observed in the aorta (Pa) over this period:

$$iFR = \frac{P_{d(wave-free\ period)}}{P_{a(wave-free\ period)}}$$

A quantitative flow ratio (QFR) may be based on 3D reconstruction of the coronary vessel and estimated contrast flow velocity derived from standard diagnostic invasive coronary angiography (ICA). QFR does not require pharmacological induction of hyperemia and invasive physiological measurements. It can be computed within the time of conventional FFR measurement.

A Resting Full-Cycle Ratio or Relative Flow Reserve (RFR) may express the ratio of the pressure at the resting diastolic pressure (Pd) compared to aortic pressure (Pa) during the cardiac cycle. It can be defined also as the ratio of hyperemic myocardial blood flow (MBF) in a stenotic area to hyperemic MBF in a normally perfused area.

A relative fractional flow reserve may be numerically calculated using Computational Fluid Dynamics (CFD) methods. It expresses the ratio of FFR calculated for the model with narrowing to the FFR calculated for the reconstructed model, measured in the same places of the coronary branch, in distal section to the narrowing. It can be simply calculated as a relative pressure drop for stenoted (P$_{sten}$) and reconstructed (P$_{rec}$) models, in distal section to the stenosis:

$$FFR_{VCAST} = \frac{FFR_{sten}}{FFR_{rec}} = \frac{\left(\frac{P_d}{P_a}\right)_{sten}}{\left(\frac{P_d}{P_a}\right)_{rec}} = \frac{(P_d)_{sten}}{(P_d)_{rec}}$$

wherein: FFR is Fractional flow reserve (FFR), usually determined as the ratio of the average pressure measured after the stenosis ($P_d$) in the distal section of the vessel to the average pressure in the aorta ($P_a$) in the conditions of maximum hyperemia.

An energy flow reference index may be numerically calculated using CFD methods. It expresses the ratio of flow energy calculated for the stenoted model to the flow energy calculated for the reconstructed model measured in the same cross area of the coronary branch, in distal section to the narrowing. It can be simply calculated as relative total pressure drop for stenoted (sten) and reconstructed (rec) models, in distal section to the stenosis:

$$EFR_{VCAST} = \frac{\left(\frac{EF_d}{EF_a}\right)_{sten}}{\left(\frac{EF_d}{EF_a}\right)_{rec}} = \frac{(EF_d)_{sten}}{(EF_d)_{rec}} = \frac{(P_{total\_d} * Q_d)_{sten}}{(P_{total\_d} * Q_d)_{rec}} = \frac{(P_{total\_d})_{sten}}{(P_{total\_d})_{rec}}$$

wherein $\left(\frac{EF_d}{EF_a}\right)$ is a relative energy drop for reconstructed (rec) and stenoted (sten) models.

The $FFR_{VCAST}$ and $EFR_{VCAST}$ may be calculated assuming the same inlet flow energy (pressure and mass flow) and the same flow rate distribution at the outlet, for both normal (reconstructed) and stenoted models;

$$(EF_a)_{rec} = (EF_a)_{sten} = EF_a$$

$$(Q_i)_{rec} = (Q_i)_{sten} = Q_i$$

Additionally, for learning process, other hemodynamic parameters can be used, which depends on the size of flow disturbances close to narrowing, caused by vessel stenosis and can be specified by a functions of pressure (stress) and flow rate, calculated from the comparative numerical simulation of both models. Among them, the following can be used: absolute and relative indexes of: vascular resistance (R), turbulent kinetic energy (TKE), pressure drop (DP), wall shear stress (WSS), oscillatory shear index (OSI) or relative residence time (RRT).

The clinical examination data are necessary for the training process to determine whether the stenosis is significant or not significant. For example, a coronary stenosis may be defined as significant if:

- the value of FFR is smaller than 0.80; and/or
- the value of iFR is smaller than 0.90; and/or
- the value of RFR is smaller than 0.89.

Otherwise, the stenosis may be determined as non-significant.

Moreover, a borderline value may be defined, for example for FFR values between 0,75 and 0,8.

Therefore, in a simple configuration, the reasoning module may output two simple parameters: significant (S) or non-significant (NS) that describe the stenosis. Optionally, a borderline (B) parameter may be defined.

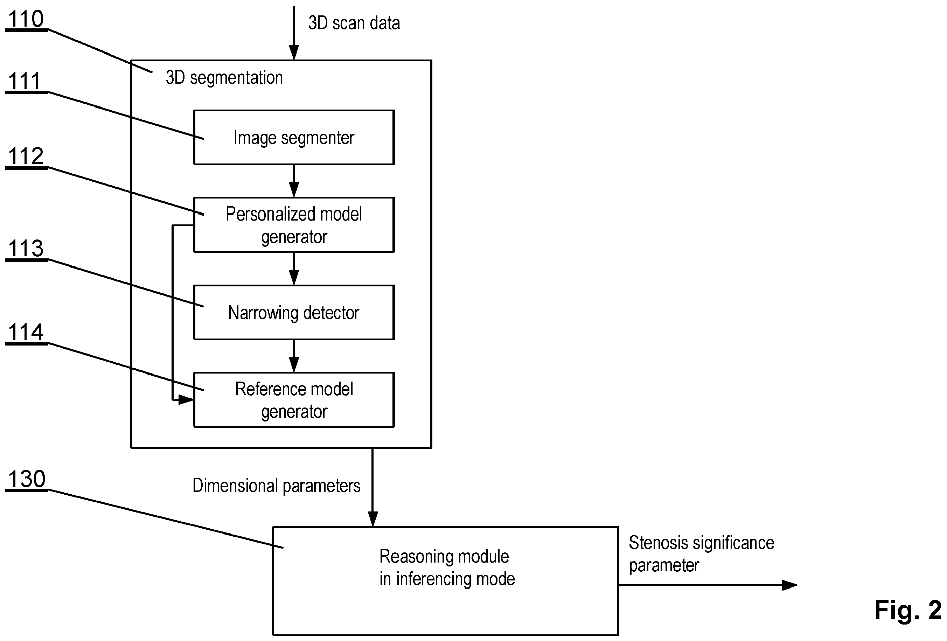

Fig. 2 shows a functional diagram of the system in accordance with an embodiment of the invention in its first inference configuration. It differs from the configuration of Fig. 1 in that the reasoning module 130 is in inference mode, wherein it has only one type data input: dimensional parameters of the blood vessel containing the stenosis, without the blood flow parameters and invasive clinical measurement data input.

In the inference mode, the reasoning module 130 operates to determine the value of the stenosis parameter at least as significant (S) or non-significant (NS). Optionally, the stenosis parameter may indicate a borderline (B) value.

Optionally, the reasoning module 130 may output additional data, such as a reliability parameter that indicates how reliable is the inference result.

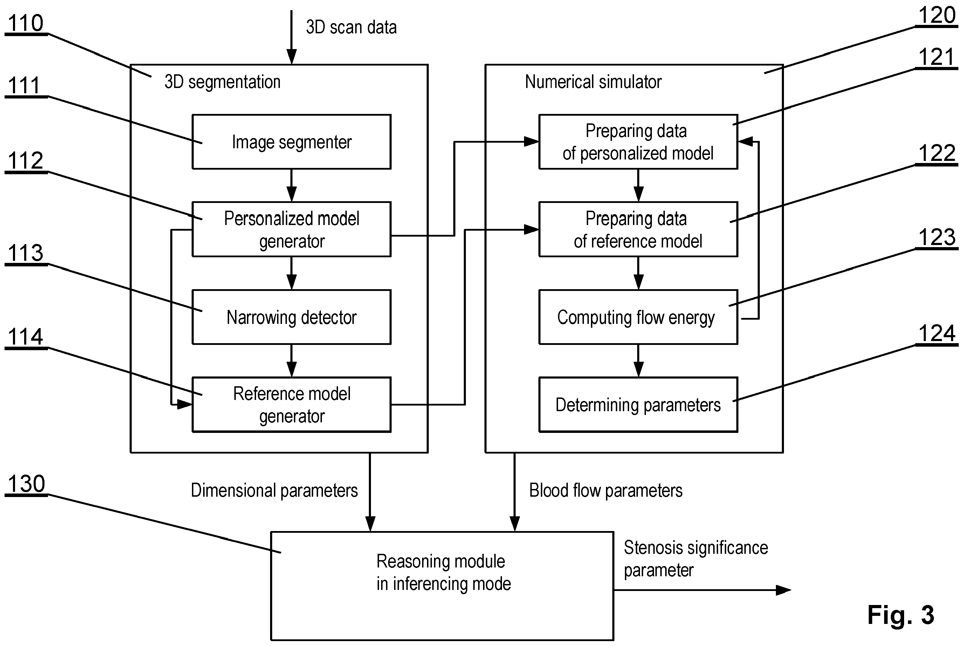

Fig. 3 shows a functional diagram of the system in accordance with an embodiment of the invention in its second inference configuration. It differs from the configuration of Fig. 1 in that the reasoning module 130 is in inference mode, wherein it has only two types of data inputs: dimensional parameters and blood flow parameters of the blood vessel containing the stenosis, without the invasive clinical measurement data input.

In the inference mode, the reasoning module 130 operates to determine the value of the stenosis parameter at least as significant (S) or non-significant (NS). Optionally, the stenosis parameter may indicate a borderline (B) value.

While the invention has been described with respect to a limited number of embodiments, it will be appreciated that many variations, modifications and other applications of the invention may be made. Therefore, the claimed invention as recited in the claims that follow is not limited to the embodiments described herein.

**Claims**

1. A method for determining a significance of a stenosis in a currently examined blood vessel, the method comprising:

    - providing a pre-trained reasoning module (130) that has been trained to output a value of a stenosis significance parameter by means of a training data set comprising a plurality of records of prior clinically examined stenosis cases, each training record comprising data related to dimensional parameters, blood flow parameters and clinical measurement parameters of the prior clinically examined blood vessel containing the stenosis;
    - inputting, to the pre-trained reasoning module (130), an examination record comprising data related to the dimensional parameters of the currently examined blood vessel containing the stenosis and instructing the reasoning module (130) to output the value of the stenosis significance parameter based on the examination record.

2. The method according to claim 1, wherein the examination record further comprises data related to the blood flow parameters of the currently examined blood vessel containing the stenosis.

3. The method according to any of previous claims, wherein the dimensional parameters of the prior clinically examined blood vessel and/or of the currently examined blood vessel include at least one of: a length of the stenosis, a thickness of the stenosis, a shape index of the stenosis, a symmetry index of the stenosis, a cross-section area of a lesion coronary, an area severity of the stenosis, a stenosed vessel volume, a reconstructed vessel volume lumen, a volume severity of the stenosis, an aggregate volume of the stenosis, an aggregate volume severity of the stenosis, a coronary score lesion grade, a vascular index, an extent score, a calcium score, a density factor, a localization of the stenosis.

4. The method according to any of previous claims, wherein the blood flow parameters of the prior clinically examined blood vessel and/or of the currently examined blood vessel include at least one of: relative fractional flow reserve ($FFR_{VCAST}$), energy flow reference index ($EFR_{VCAST}$), a vascular resistance (R), a turbulent kinetic energy (TKE), a pressure drop (DP), a wall shear stress (WSS), oscillatory shear index (OSI), a relative residence time (RRT).

5. The method according to any of previous claims, wherein the clinical measurement parameters of the prior clinically examined blood vessel include at least one of: a fractional flow reserve (FFR), an instantaneous wave-free ratio (iFR), a resting full-cycle ratio or relative flow reserve (RFR).

6. The method according to any of claims 1-5, wherein the reasoning module (130) is a convolutional neural network.

7. The method according to any of previous claims, further comprising providing an image segmenter (111) implemented as a neural network trained to process input image data and to output a 3D segmented model containing description of blood vessels arrangement within the imaged volume, including at least the location and dimensions of the vessels, wherein the image segmenter (111) is trained by performing the following steps:

    - prediction of output binary mask based on the input CT imaging data;
    - the computation of the difference between the ground truth mask (as given in the training data) and the predicted mask;
    - the update of weights according to the gradient backpropagation method.

8. The method according to any of claims 1-6, further comprising providing an image segmenter (111) configured to process input image data and to output a 3D segmented model containing description of blood vessels arrangement within the imaged volume, including at least the location and dimensions of the vessels by use of at least one of the following half-automated or fully-automated methods:

    - region growing from seed,
    - active shapes and active contours,
    - segmentation based on graph cuts,
    - adaptive thresholding,

- segmentation based on rough sets,
- watershed segmentation,
- vesselness filters (Frangi, Jerman),
- connectedness methods (fuzzy, Bayesian).

9. A computer-implemented system comprising:

- a pre-trained reasoning module (130) that has been trained to output a value of a stenosis significance parameter by means of a training data set comprising a plurality of records of prior clinically examined stenosis cases, each training record comprising data related to dimensional parameters, blood flow parameters and clinical measurement parameters of the prior clinically examined blood vessel containing the stenosis;
- wherein the pre-trained reasoning module (130) has an input configured to receive an examination record comprising data related to the dimensional parameters of the currently examined blood vessel containing the stenosis and an output configured to provide the value of the stenosis significance parameter based on the examination record.

Fig. 1

Fig. 2

Fig. 3

EP 3 884 848 A1

Fig. 4

Europäisches
Patentamt

European
Patent Office

Office européen
des brevets

# EUROPEAN SEARCH REPORT

Application Number

EP 20 16 4757

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2019/318476 A1 (ISGUM IVANA [NL] ET AL) 17 October 2019 (2019-10-17) * paragraphs [0060] - [0070], [0077] - [0091], [0096] - [0123], [0129] - [0152]; claims; figures * | 1-9 | INV. A61B5/00 A61B5/02 ADD. A61B6/00 |
| X | US 2018/315505 A1 (ITU LUCIAN MIHAI [RO] ET AL) 1 November 2018 (2018-11-01) * paragraphs [0064] - [0084] * | 1-9 | |
| X | US 2015/282765 A1 (GOSHEN LIRAN [IL] ET AL) 8 October 2015 (2015-10-08) * paragraphs [0021] - [0069] * | 1-9 | |
| X | MAJD ZREIK ET AL: "Deep learning analysis of the myocardium in coronary CT angiography for identification of patients with functionally significant coronary artery stenosis", ARXIV.ORG, CORNELL UNIVERSITY LIBRARY, 201 OLIN LIBRARY CORNELL UNIVERSITY ITHACA, NY 14853, 24 November 2017 (2017-11-24), XP081302471, DOI: 10.1016/J.MEDIA.2017.11.008 * the whole document * | 1-9 | |

| TECHNICAL FIELDS SEARCHED (IPC) |
|---|
| A61B G06T G16H |

The present search report has been drawn up for all claims

| Place of search | Date of completion of the search | Examiner |
|---|---|---|
| The Hague | 22 September 2020 | Crisan, Carmen-Clara |

EPO FORM 1503 03.82 (P04C01)

**ANNEX TO THE EUROPEAN SEARCH REPORT
ON EUROPEAN PATENT APPLICATION NO.**                    EP 20 16 4757

This annex lists the patent family members relating to the patent documents cited in the above-mentioned European search report.
The members are as contained in the European Patent Office EDP file on
The European Patent Office is in no way liable for these particulars which are merely given for the purpose of information.

22-09-2020

| Patent document cited in search report | | Publication date | Patent family member(s) | | Publication date |
|---|---|---|---|---|---|
| US 2019318476 | A1 | 17-10-2019 | US 2019318476 A1<br>WO 2019197450 A1 | | 17-10-2019<br>17-10-2019 |
| US 2018315505 | A1 | 01-11-2018 | NONE | | |
| US 2015282765 | A1 | 08-10-2015 | BR 112015010012 A2<br>CN 104768465 A<br>EP 2916735 A2<br>JP 6302922 B2<br>JP 2016500548 A<br>RU 2015121362 A<br>US 2015282765 A1<br>WO 2014072861 A2 | | 11-07-2017<br>08-07-2015<br>16-09-2015<br>28-03-2018<br>14-01-2016<br>27-12-2016<br>08-10-2015<br>15-05-2014 |

For more details about this annex : see Official Journal of the European Patent Office, No. 12/82

EPO FORM P0459

## REFERENCES CITED IN THE DESCRIPTION

**Patent documents cited in the description**

- EP 19174972 A